**Europäisches Patentamt**

**European Patent Office**

**Office européen des brevets**

(11) Veröffentlichungsnummer: **0 310 757 B1**

# EUROPÄISCHE PATENTSCHRIFT

(45) Veröffentlichungstag der Patentschrift: **18.12.91**

(51) Int. Cl.⁵: **A61K 35/64**

(21) Anmeldenummer: **88109824.8**

(22) Anmeldetag: **21.06.88**

(54) **Verfahren zur Herstellung eines Propolis-Äthanolextraktes.**

(30) Priorität: **05.10.87 YU 1835/87**

(43) Veröffentlichungstag der Anmeldung:
**12.04.89 Patentblatt 89/15**

(45) Bekanntmachung des Hinweises auf die
Patenterteilung:
**18.12.91 Patentblatt 91/51**

(84) Benannte Vertragsstaaten:
**AT BE DE FR SE**

(56) Entgegenhaltungen:
**EP-A- 0 109 993**

(73) Patentinhaber: **Wagner, Vera**
**Weishamerstrasse 5**
**W-8214 Bernau(DE)**

(72) Erfinder: **Ferluga, Dusan**
**Nazorjeva 12**
**YU-66310 Izola(YU)**
Erfinder: **Mihelic, Ana Marija**
**Prelog 114**
**YU-61230 Domzale(YU)**
Erfinder: **Raktelj, Boris**
**Soncna pot 23**
**YU-66320 Portoroz(YU)**
Erfinder: **Wagner, Vera**
**Weishamerstrasse 5**
**W-8214 Bernau(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues Verfahren zur Herstellung von Propolis-Äthanolextrakten mittels Ultraschallbehandlung.

Das vorliegende Verfahren zur Ultraschallgewinnung von flüssigen Propolis-Äthanolextrakten wird derart ausgeführt, daß die auf eine Teilchengröße von 3 mm Durchmesser vermahlene Propolis bei niedriger Temperatur, 0° bis 20° C, mit einem Äthanol/Wassergemisch (vol. Verhältnis 87 : 13) durch die Anwendung eines Ultraschallsystems extrahiert wird.

Die Frequenz beträgt bei der Extraktion 18 bis 25 kHz. Die Extraktion dauert etwa 25 Minuten. Der erhaltene Propolisextrakt wird durch Dekantierung und Filtrierung von den beigemischten Wachsen befreit und ausschliessend bis zum erforderten Prozentgehalt der Trockensubstanz (max. 80 %) in einem 1471 - 490 Pa (150 - 50 mm $H_2O$) Vakuum eingeengt.

Propolis, auch Stopfwachs genannt, ist ein Naturerzeugnis der Bienen. Sie sammeln sie an den Knospen und anderen Pflanzenteilen und verwenden sie in ihrem Lebensraum zum Verstopfen von Öffnungen und Rissen sowie für die Isolierung von toten Fremdkörpern (Insekten und anderen Tieren) im Bienenstock, und verhindern damit die Infektionsausbreitung. Sie überziehen mit ihr die Wabenzellen, bevor sie darin ihre Erzeugnisse, wie z. B. den Honig und den Blütenstaub, speichern. Propolis ist eine spezifische komplexe bioaktive Substanz, welche aus über 60 verschiedenen Verbindungen besteht. Die Grundkomponenten sind verschiedene Harze, Wachse, ätherische Öle und der Blütenstaub. Bedeutende bioaktive Inhaltsstoffe der Propolis sind ausserdem pflanzliche Farbstoffe, von denen die wichtigsten die Flavone oder gelbe Farbstoffe wie Krysin oder Tektokrysin, Flavonone wie Pinostrombin, Pinocembrin und Quercetin und deren Derivate, sowie Flavonole wie Rhamnocitrin, Galangin und Isoalpinin sind. Sie enthält auch Aromastoffe wie Isovanilin, Acetoxybetulenol und aromatische Säuren wie Zimtsäure, Benzoesäure, Kaffeesäure, Ferulasäure und Protocatechusäure sowie deren Ester mit Benzylalkohol, Pentonylalkohol, Phenetylalkohol und Zimtalkohol (E. M. Schneidewind, A. Brige, H. Kala, J. Metzner und A. Zsunke - Die Pharmazie Nr. 34, 1979 - 103). Die meisten angeführten Stoffe wirken antimikrobiell und fungistatisch, was auch charakteristisch für die Wirkung der Propolis ist.

Bei der Lösung der Propolis in Äthanol wird diese harzartige, braune, thermoplastische und klebrige Masse einigermaßen gelöst. Nach einiger Zeit scheiden sich aus der Masse die Wachse aus. Nach dem bisher bekannten Verfahren (C. Braileanu et al., Die Propolis; Apimondia Verlag, Bukarest 1975, S. 147) wird der Propolisextrakt durch die Einweichung der Propolis während 10 Tagen in einem Alkohol/Wassergemisch (Alkoholgehalt 30 bis 96%) in einem Verhältnis von 1 : 10 gewonnen. Die Suspension wird zwischendurch gerührt. Nach 10 Tagen wird die Lösung abfiltriert und als solche verwendet.

Bei einer derartigen Herstellung des Propolisextraktes treten Probleme auf, wie z.B. die schwache Extrahierung, die lange Dauer des Verfahrens, die geringe Ausbeute, die Adhäsion des Harzes an die Wände des Gefäßes.und an den Rührer. Es bilden sich harzartige Aggregate, welche sich nur schwer und unvollkommen im Extraktionsmittel auflösen; darum sind auch die Extraktionsausbeuten sehr niedrig. Beim mehrtägigen Stehenlassen der Lösung an der Luft kommt es zur Oxydierung einiger Inhaltsstoffe, was die Aktivität des Endproduktes vermindert. Ein grosses Problem beim klassischen Extraktionsverfahren ist auch die schwache Auflösung der harzartigen und wachsartigen Komponenten der Propolis, was zu einer späteren Ausscheidung der Wachse und bei der folgenden Verarbeitung zum Verstopfen der Filtervorrichtungen führt.

Im Vergleich mit den bisher bekannten Methoden zur Extraktion der Propolis weist das vorliegende Verfahren bedeutende Vorteile und Neuheiten auf, wie folgt:

- die Extraktion wird bei niedrigen Temperaturen ausgeführt, was von grundlegender Bedeutung ist, um die bioaktiven, thermolabilen Stoffe nicht zu zersetzen;
- bei niedrigen Extraktionstemperaturen von 0 bis 20° C ist die Löslichkeit der Wachse im Extraktionslösungsmittel niedrig und demgemäß wird die spätere Ausflockung der Wachse stark eingeschränkt; die übrigen Aktivsubstanzen werden jedoch vollständig extrahiert;
- die Extraktionsausbeute beträgt mindestens 60 % und kann sogar über 98 % betragen, was wesentlich höher ist, als im Stand der Technik beschrieben ist;
- der Äthanolverbrauch ist wirtschaftlicher;
- die Extraktionsausführung fordert weniger Arbeitskräfte;
- die Extraktion wird auf einem kleinerem Raum als die klassische Mazeration ausgeführt, weil diese zahlreiche rostfreie Zisternen erfordert, was beim vorliegenden Verfahren vermieden wird;
- die Extraktion ist äußert wirkungsvoll, da der Ultraschall aufschluß der Zellen die Extraktion aller bioaktiven Substanzen ermöglicht, was im Vergleich mit der klassischen Mazeration einen aktiveren Propolisextrakt ergibt;

- infolge der fortschrittlichen Ausführung der Extraktion im geschlossenen System wird auch die Oxydationsanfälligkeit der Aktivsubstanzen stark vermindert, wodurch die Möglichkeit, einen hochwirksamen Extrakt zu gewinnen, gegeben ist;
- die Ultraschallextraktion der Propolis ergibt ein Extrakt, welcher einen hohen Gehalt an bioaktiven Flavonen aufweist;
- es wurde die hohe antimikrobielle Aktivität des Propolisextraktes, welcher gemäß der voliegenden Ultraschallextraktion erhälten wurde bewiesen, was u. E. durch den hohen Flavonoidengehalt bedingt ist.

Aufgrund der bisherigen Forschung wurde gefunden, daß:
- der Propolisextrakt bei lokaler Applikation eine antibakterielle Wirkung auf ein breites Bakterienspektrum, insbesondere von Gram-negativen Bakterien, welche Haut- und Schleimhautentzündungen verursachen, ausübt;
- inhibierend auf das Wachstum von verschiedenen Pilzen wirkt;
- eine ausgeprägte lokal-anästhetische Wirksamkeit aufweist;
- die Regenerierung der Hautepithelzellen bei lokaler Behandlung beschleunigt;
- antiinflammatorisch (entzündungshemmend) bei lokaler Applikation wirkt;
- virozid wirkt (auf den Herpes simplex Virus, auf den Influenza virus).

Dokumentation:
- L. Vechet: Die Propolis, Verlag Apimondia, Bukarest 1975, S. 48-52
- J. Metzner, E. Schneidewind und E. Friedrich: Die Pharmazie, 32/1977, S. 730
- J. Metzner, H. Bekemeier, M. Paintz und E. Schneidewind: Die Pharmazie, 34/1979, S. 97
- M. Paintz und J. Metzner: Die Pharmazie, 34/1979, S. 839
- A. Stojko et al.: Arzneimitt. Forsch. - Drug. Res., 28/1977, S. 35
- S. Scheller et al.: Arzneimitt. Forsch. - Drug. Res., 27/1979, S. 1547
- M. Likar und B. Filipič: Die Erforschung der antimikrobiellen Aktivität des Propolis - Äthonolextraktes, ausgefürt an der Medizinischen Fakultät, Institut für Mikrobiologie, in Ljubljana, J. 1979 bis 1986.

Aufgrund der obigen Dokumentation und der Resultate von zahlreichen biologischen und klinischen Untersuchungen in der Dermetalogie, Stomatologie und Otorhinolaryungologie, kann der Propolis-Äthanolextrakt bei Entzündungen der Mundhöhle und des Rachens, bei lokalen Behandlungen von kleinen Wunden, Geschwüren, und Abschürfungen, sowie bei der Prävention des Organismus gegen einige Vireninfektionen (Influenza, Herpes) sowie in Salbengrundlagen eingebettet als entzündungshemmendes Mittel (bei Sonnen- und Quallenbränden) eingesetzt werden.

Der Propolisextrakt wird in der Form einer 5- bis 80%-iger Lösung in einem Äthanol/Wassergemisch (vol. Verhältnis 60 : 40 bis 96 : 4) verwendet. Bei lokaler Verabreichung wird der Propolis-Äthanolextrakt in der Form eines 5 bis 80%-igen Propolis-Äthanolextraktes oder in Salbenform eingesetzt. In Tabletten Kapsel- oder Drageeform ist er für die orale oder sublinguale Applikation oder in Suppositorienform für die rektale Applikation geeignet. Beim Schutz gegen Infektionen wird der Propolisextrakt mit Honig vermengt und mit Gelee Royale und Blüttenstaub kombiniert angewandt.

Die Erfindung soll durch die folgenden Beispiele erläutert werden.

Ausführungsbeispiele

Beispiel 1 (Herstellung des Extraktes)

Die Propolis wird in einer geeigneten Mühle auf feine Teilchen (max. Durchmesser 5 mm) gemahlen.

Im Extraktionsgefäss wird 500 Liter einer min. 87%-iger (Gew.) Äthanollösung angemacht. Unter Rühren werden in den Extraktor 20 kg der gemahlenen Propolis eingetragen. Der Aussatz wird 25 Minuten einer Ultraschallextraktion (18 - 25 kHz) unterworfen, auschliessend wird 2 Stunden stehengelassen, dekantiert und filtriert.

Die Filtrierung wird über einem Preßfilter mit Rapid-Filterpapiereinlagen ausgeführt. Der erhaltene klare Propolisextrakt wird in einem Säulenkonzentrator in einem 1471 - 490 Pa (150 - 50 mm $H_2O$) Vakuum mittels einer Wärmepumpe bis zur geforderten Konzentration 5 bis 80 % der Trockensubstanz im Extrakt eingeengt. Die Konzentrierung wird bei einer Temperatur von max. 20° C ausgeführt.

| Beispiel 2 (Tablettenzusammensetzung) | |
|---|---|
| Inhaltsstoffe | mg pro Tablette |
| Propolisextrakt 80%-ig | 17,5 |
| Vitamin C | 1,5 |
| Menthae piperitae Öl | 0,7 |
| Saccharose | 681,3 |
| | 700 mg |

| Beispiel 3 (Salbenzusammensetzung) | |
|---|---|
| Inhaltsstoffe | g pro 100 g der Salbe |
| Propolisextrakt 80%-ig | 5 |
| Neutrale Salbengrundlage | 95 |
| | 100 g |

**Patentansprüche**

1. Verfahren zur Herstellung eines Propolis-Äthanolextraktes, dadurch gekennzeichnet, daß die Propolis bei einer niedrigen Temperatur, wie max 20 ° C, mit einem Äthanol/Wassergemisch in einem vol. Verhältnis von 87 : 13 unter Ultraschallbehandlung von 18 bis 25 kHz kurzzeitig wie 25 Minuten, in einem geschlossenen System extrahiert wird, und anschließend die erhaltene Suspension dekantiert und der klare Propolisextrakt von den festen Teilchen befreit wird.

**Claims**

1. A process for the preparation of an ethanol extract of propolis, **characterised in that** the propolis is extracted in a closed system by subjecting to a short-time, e.g. 25 minutes ultrasonic treatment at 18 to 25 kHz, at a low temperature, such as maximum 20 ° C, with an ethanol-water mixture of a vol. ratio of 87:13, and subsequently the obtained suspension is decanted, whereupon there are separated the solid particles and the clear propolis extract.

**Revendications**

1. Procédé de préparation d'un extrait éthanolique de propolis, **caractérisé en ce qu'**on extrait la propolis dans un système clos par traitement ultrasonique à 18 jusqu'à 25 kHz, de brève, durée, à savoir 25 minutes, à une température réduite, à savoir 20 ° C au maximum, par ur mélange de l'éthanol et de l'eau ayant un rapport volumétrique de 87:13, suivi par la décantation de la suspension obtenue et la séparation des particules solides et de l'extrait clair de propolis.